(19) Europäisches Patentamt
European Patent Office
Office européen des brevets

(11) **EP 4 373 584 B1**

(12) **EUROPEAN PATENT SPECIFICATION**

(45) Date of publication and mention
of the grant of the patent:
**19.03.2025 Bulletin 2025/12**

(21) Application number: **22751367.8**

(22) Date of filing: **18.07.2022**

(51) International Patent Classification (IPC):
*A61Q 11/00* (2006.01)    *A61K 8/25* (2006.01)
*A61K 8/73* (2006.01)

(52) Cooperative Patent Classification (CPC):
**A61Q 11/00; A61K 8/25; A61K 8/73;** A61K 2800/28

(86) International application number:
**PCT/EP2022/070094**

(87) International publication number:
**WO 2023/001777 (26.01.2023 Gazette 2023/04)**

(54) **AN ORAL CARE COMPOSITION**

MUNDPFLEGEZUSAMMENSETZUNG

COMPOSITION DE SOIN ORAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.07.2021 EP 21186818**

(43) Date of publication of application:
**29.05.2024 Bulletin 2024/22**

(73) Proprietors:
• **Unilever IP Holdings B.V.**
**3013 AL Rotterdam (NL)**
Designated Contracting States:
**AL AT BE BG CH CZ DK EE ES FI FR GR HR HU IS
LI LT LU LV MC MK NL NO PL PT RO SE SI SK SM**
• **Unilever Global IP Limited**
**Wirral, Merseyside CH62 4ZD (GB)**
Designated Contracting States:
**CY DE GB IE IT MT RS TR**

(72) Inventor: **JOINER, Andrew**
**6708 WH Wageningen (NL)**

(74) Representative: **Tansley, Sally Elizabeth**
**Unilever Patent Group**
**Bronland 14**
**6708 WH Wageningen (NL)**

(56) References cited:
**WO-A1-2019/025180    JP-B2- 5 168 465
US-A- 6 162 418**

• "Non-Aqueous Toothpaste Containing Ion-Exchange Carrageenan ED  - Darl Kuhn", IP.COM, IP.COM INC., WEST HENRIETTA, NY, US, 30 April 2020 (2020-04-30), XP013186497, ISSN: 1533-0001
• SUBRAMANIAN SANGEETHA ET AL: "The Role of Abrasives in Dentifrices", JOURNAL OF PHARMACEUTICAL SCIENCES AND RESEARCH, vol. 9, no. 2, 1 February 2017 (2017-02-01), IN, pages 221 - 224, XP055975369, ISSN: 0975-1459, Retrieved from the Internet <URL:https://www.jpsr.pharmainfo.in/Documents/Volumes/vol9Issue02/jpsr09021730.pdf>

**Description**

**Field of the Invention**

The present invention relates to an oral care composition for minimising staining of teeth.

**Background of the Invention**

Long-lasting whitening of teeth is of considerable interest to consumers. Foods and drinks such as tea, coffee and wine may form dental stains by directly depositing chromogens on the tooth surface. Attraction of materials to the tooth surface plays a critical role in the deposition of extrinsic dental stain. The chromogens in these beverages that are responsible for causing dental stain are known as tannins and are composed of polyphenols such as catechins. These materials generate colour due to the presence of conjugated double bonds.

Traditional tooth whitening methods involve either peroxide bleaching from kit formats or abrasive stain removal from toothpaste formats. These particles are essentially insoluble particles that remove extrinsic stain from the surface of the tooth via abrasion when applied with a brush. The present invention relates to a novel oral care composition that delivers effective levels of stain removal.

US2003124065A (P&G) discloses an oral care composition and methods for overall cleaning, whitening and preventing, reducing or removing surface deposited stains on natural teeth and dental prosthesis, the compositions comprising in an orally acceptable carrier at least 0.1% by weight of a water-soluble or water-dispersible copolymer prepared by copolymerizing one or a mixture of vinyl pyrrolidone (VP) monomers with one or a mixture of C1-C19 alkyl carboxylic acid (AC) C2-C12 alkenyl ester monomers; preferably, these compositions further comprise one or a mixture of other oral care agents selected from a water soluble alkali metal or ammonium tripolyphosphate in an amount at least about 0.5% by weight of the composition, an abrasive, preferably a precipitated silica abrasive, in an amount at least about 6% by weight of the composition and a bleaching agent in an amount at least about 0.1% by weight of the composition.

It is thus an object of the present invention to provide for an oral care composition that removes the staining of teeth and increases whitening.

**Description of the Invention**

A first aspect of the invention relates to a toothpaste composition comprising a silica abrasive, an acid anhydride polymer and a carrageenan consisting of 33wt% to 85wt% of the total level of carrageen of iota carrageenan and 15 wt% to 67 wt% of the total level of carrageenan kappa carrageenan.

A second aspect of the invention relates to a method of removing the stains from teeth comprising the steps of applying the composition as described above on to a tooth surface.

A third aspect of the invention relates to a method of whitening the teeth comprising the steps of applying the composition as described above to a tooth surface.

According to another aspect of the present invention there is provides the cosmetic use of a composition as described above to whiten the teeth.

**Detailed Description of the Invention**

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Compositions according to the invention comprise carrageenan. The carrageen present in the composition of the invention consists of from 33wt% to 85wt% of the total level of iota carrageenan and 15 wt% to 67 wt% of kappa carrageenan.

Preferably the level of iota carrageenan is from 40 wt% to 60 wt% of the total level of carrageenan. Preferably the level of kappa carrageenan is from carrageenan is from 40 wt% to 60 wt% of the total level of carrageenan.

The total level of carrageenan is from 0.1 to 5 wt%, preferably 0.3 to 3 wt% of the total composition.

Compositions of the invention comprise a silica abrasive. usually in amounts between 3 and 60% by weight of the oral care composition. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 $m^2$/g and an oil absorption of about 70 - 150 $cm^3$/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5-60% by weight, usually 5-20% by weight.

Compositions according to the invention may comprise a whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

In one preferred embodiment of the invention the whitening system comprises a combination of green and blue pigment, the weight ratio of green pigment to blue pigment is greater than 1:2, preferably greater than 2:3 most preferably the weight ratio of green pigment to blue pigment is from 2:3 to 3:2.

Preferably the total level of pigment in the composition is from 0.01 wt% to 3 wt, more preferably from 0.02 to 2 wt%.

If the composition is a toothpaste it may be a dual phase paste, with , if present, the whitening pigments present in one phase.

Compositions according to the invention comprise a polymeric deposition aid which comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:

Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions of the invention are preferably toothpastes.

Preferably the compositions comprise greater than 25 wt% of the total composition 30 wt% of water.

The composition may comprise an inorganic or a natural or synthetic thickener or gelling agent in proportions of about 0.10 to about 15% by weight of the total composition depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, tragacanth, and polyvinylpyrrolidone. In the context of the present invention silica is particularly preferred.

Suitable foaming agents for use in forming the composition include surfactants. Surfactants help to increase the rate of dissolution of the solid oral care composition when in contact with saliva, and assist in foaming of the composition during usage.

Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Preferred surfactants for use in the invention include those anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

Mixtures of any of the above described materials may also be used.

The total amount of surfactant incorporated into the composition of the invention generally ranges from about 0.2 to about 5%, by total weight surfactant based on the total weight of the composition.

The toothpaste composition will comprise further ingredients which are common in the art, such as:

antimicrobial agents, e.g. chlorhexidine, sanguinarine extract, metronidazole, quatemary ammonium compounds, such as cetylpyridinium chloride cetylpyridium chloride clay complex ; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;

Humectants such as glycerol, sorbitol, propyleneglycol, xylitol, lactitol etc.;

binders and thickeners such as sodium carboxymethyl-cellulose, hydroxyethyl cellulose (Natrosol®), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Example of the invention will now be illustrated by the following non-limiting examples:

Examples

Table 1: Base Paste Formulation

| Ingredient | % w/w |
|---|---|
| Water | To 100 |

(continued)

| Ingredient | % w/w |
|---|---|
| Sorbitol | 45.00 |
| Sodium Fluoride | 0.32 |
| Sodium saccharin | 0.5 |
| Gantrez S-97 | 0.052 |
| Silica Thickening | 8.00 |
| Silica Abrasive | 9.00 |
| carrageenan | 0.75 |
| Sodium lauryl sulphate | 1.70 |
| Silica granules | 0.30 |
| Carrageenan* Example A 25 wt% iota, 75wt% kappa Example 1 50 wt% iota, 50 wt% kappa Example 2 75 wt% iota, 25 wt% kappa Example B 100% iota | |

Hydroxyapatite (HAP) discs were cleaned and polished. The baseline (**initial clean)** colour was measured using a chromameter (Minolta CR200 series). Tannic acid (0.5% w/w) dissolved in Milli Q water and Ammonium iron II sulphate hexahydrate (0.5% w/w) dissolved in Milli Q water were prepared and the solutions were mixed in the ratio of 1:1 by volume to form the FT stain solution and used immediately. The FT stain solution was applied to the HAP discs using a small watercolour paintbrush and left to air dry (approximately 15mins). A further 3 to 4 coats of the stain were applied to each disc and allowed to dry for about 30 minutes. Sufficient stain had been applied once the L* reading was <56. The discs were then left for ~45 mins before remeasuring the colour (soiled). The HAP disks were brushed with the toothpastes for 2 minutes and then for an additional 3 minutes to get a total of 5 minutes of brushing at a speed of 150 cycles per min. The HAP discs were removed from the well, rinsed with water and gently dried using a soft tissue and the colour was remeasured.

The % stain removal was calculated for each HAP disc using the equation below and the mean % stain removal determined.

$$\% \text{ stain removal} = \frac{L^* \text{(cleaned)} - L^* \text{(soiled)}}{L^* \text{(initial clean)} - L^* \text{(soiled)}} \times 100$$

The results are shown in table 2

### Table 2: Mean % Stain removal after 2 and 5 minutes brushing

| Example | 25% Iota A | 50% Iota 1 | 75% Iota 2 | 100% Iota B |
|---|---|---|---|---|
| 2 min | 34.03 | 44.31 | 38.3 | 24.93 |
| 5 min | 57.13 | 65.2 | 59.74 | 45.04 |

The results show that Examples (1 and 2) according to the invention remove stains more effectively than the comparative Examples (A and B).

## Claims

1. A toothpaste composition comprising a

   i) silica abrasive;
   ii) an acid anhydride polymer
   iii) a carrageenan consisting of 33wt% to 85wt% of the total level of carrageen of iota carrageenan and 15 wt% to 67 wt% of the total level of carrageenan kappa carrageenan.

2.  A toothpaste composition according to claim 1 in which the level of iota carrageenan is from 40 wt% to 60 wt% of the total level of carrageenan.

3.  A toothpaste composition according to any preceding claim in which the level of kappa carrageenan is from carrageenan is from 40 wt% to 60 wt% of the total level of carrageenan.

4.  A toothpaste composition according to any preceding claim in which the level of carrageenan is from 0.1 to 5 wt%, preferably 0.3 to 3 wt% of the total composition.

5.  A toothpaste composition according to any preceding claim in which the acid anhydride polymer is a co-polymers of maleic anhydride with methyl vinylether, in which the anhydride moiety may be in a partially or fully hydrolysed or alcoholysed form.

6.  A toothpaste composition according to any preceding claim that comprises greater than 30 wt% of water.

7.  A toothpaste composition according to any preceding claim in which the level of silica abrasive is from 5 wt% to 60 wt% of the total composition.

8.  A composition according to any preceding claim which further comprises a polyhydric alcohol preferably sorbitol or glycerol.

9.  An oral care composition according to any preceding claim that further comprises an anionic surfactant.

10. A non-therapeutic method of removing the stains from teeth comprising the steps of applying the composition as claimed in any one of the preceding claims on to a tooth surface.

11. A non-therapeutic method of whitening the teeth comprising the steps of applying the composition as claimed in any one of the claims 1 to 9 on to a tooth surface.

12. Non-therapeutic cosmetic use of a composition according to any one of claims 1 to 9 to whiten the teeth.


**Patentansprüche**

1.  Zahnpastazusammensetzung, umfassend

    i) ein Siliziumdioxid-Schleifmittel;
    ii) ein Säureanhydrid-Polymer;
    iii) ein Carrageen, bestehend zu 33 bis 85 Gew.-% des gesamten Carrageengehalts aus Iota-Carrageen und zu 15 bis 67 Gew.-% des gesamten Carrageengehalts aus Kappa-Carrageen.

2.  Zahnpastazusammensetzung nach Anspruch 1, in welcher der Gehalt des Iota-Carrageens 40 bis 60 Gew.-% des gesamten Carrageengehalts beträgt.

3.  Zahnpastazusammensetzung nach einem vorhergehenden Anspruch, in welcher der Gehalt des Kappa-Carrageens 40 bis 60 Gew.-% des gesamten Carrageengehalts beträgt.

4.  Zahnpastazusammensetzung nach einem vorhergehenden Anspruch, in welcher der Gehalt des Carrageens 0,1 bis 5 Gew.-%, bevorzugt 0,3 bis 3 Gew.-% der Gesamtzusammensetzung beträgt.

5.  Zahnpastazusammensetzung nach einem vorhergehenden Anspruch, in welcher das Säureanhydrid-Polymer ein Copolymer von Maleinsäureanhydrid mit Methylvinylether ist, wobei der Anhydridanteil in teilweise oder vollständig hydrolysierter oder alkoholisierter Form vorliegen kann.

6.  Zahnpastazusammensetzung nach einem vorhergehenden Anspruch, die mehr als 30 Gew.-% Wasser umfasst.

7.  Zahnpastazusammensetzung nach einem vorhergehenden Anspruch, in welcher der Gehalt des Siliziumdioxid-Schleifmittels 5 bis 60 Gew.-% der Gesamtzusammensetzung beträgt.

**8.** Zahnpastazusammensetzung nach einem vorhergehenden Anspruch, die außerdem einen mehrwertigen Alkohol, vorzugsweise Sorbitol oder Glycerin, umfasst.

**9.** Mundpflegezusammensetzung nach einem vorhergehenden Anspruch, welche außerdem ein anionisches Tensid umfasst.

**10.** Nicht-therapeutisches Verfahren zum Entfernen von Flecken von Zähnen, welches den Schritt des Auftragens der Zusammensetzung, wie in irgendeinem der vorhergehenden Ansprüche beansprucht, auf eine Zahnoberfläche umfasst.

**11.** Nicht-therapeutisches Verfahren zum Aufhellen der Zähne, welches den Schritt des Auftragens der Zusammensetzung, wie in irgendeinem der Ansprüche 1 bis 9 beansprucht, auf eine Zahnoberfläche umfasst.

**12.** Nicht-therapeutische kosmetische Verwendung einer Zusammensetzung nach irgendeinem der Ansprüche 1 bis 9 zum Aufhellen der Zähne.


**Revendications**

**1.** Composition de pâte dentifrice comprenant

i) un abrasif à base de silice;
ii) un polymère d'anhydride d'acide
iii) un carraghénane consistant en 33 % en poids à 85 % en poids de la teneur totale en carraghénane en carraghénane iota et en 15 % en poids à 67 % en poids de la teneur totale en carraghénane en carraghénane kappa.

**2.** Composition de pâte dentifrice selon la revendication 1, dans laquelle la teneur en carraghénane iota est de 40 % en poids à 60 % en poids de la teneur totale en carraghénane.

**3.** Composition de pâte dentifrice selon l'une quelconque des revendications précédentes, dans laquelle la teneur en carraghénane kappa est de 40 % en poids et 60 % en poids de la teneur totale en carraghénane.

**4.** Composition de pâte dentifrice selon l'une quelconque des revendications précédentes, dans laquelle la teneur en carraghénane est de 0,1 à 5 % en poids, de préférence de 0,3 à 3 % en poids de la composition totale.

**5.** Composition de pâte dentifrice selon l'une quelconque des revendications précédentes, dans laquelle le polymère d'anhydride d'acide est un copolymère d'anhydride maléique avec du méthylvinyléther, dans lequel la fraction anhydride peut être sous une forme partiellement ou totalement hydrolysée ou alcoolysée.

**6.** Composition de pâte dentifrice selon l'une quelconque des revendications précédentes, qui comprend plus de 30 % en poids d'eau.

**7.** Composition de pâte dentifrice selon l'une quelconque des revendications précédentes, dans laquelle la teneur en abrasif à base de silice est de 5 % en poids à 60 % en poids de la composition totale.

**8.** Composition selon l'une quelconque des revendications précédentes, qui comprend en outre un alcool polyhydrique, de préférence le sorbitol ou le glycérol.

**9.** Composition de soins bucco-dentaires selon l'une quelconque des revendications précédentes, qui comprend en outre un tensioactif anionique.

**10.** Procédé non thérapeutique d'élimination des taches des dents comprenant les étapes d'application de la composition selon l'une quelconque des revendications précédentes sur une surface dentaire.

**11.** Procédé non thérapeutique de blanchiment des dents comprenant les étapes d'application de la composition telle que revendiquée dans l'une quelconque des revendications 1 à 9 sur une surface dentaire.

12. Utilisation cosmétique non thérapeutique d'une composition selon l'une quelconque des revendications 1 à 9 pour blanchir les dents.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003124065 A

- EP 236070 A